# EUROPÄISCHE PATENTSCHRIFT

(11) **EP 2 863 887 B1**
(45) Veröffentlichungstag und Bekanntmachung des Hinweises auf die Patenterteilung: **26.09.2018**
(21) Anmeldenummer: 13759658.1
(22) Anmeldetag: 20.06.2013
(51) Int. Cl.: A61K 47/10, A61K 47/24, A61K 47/26, A61K 31/10, A61K 9/02, A61K 47/34, A61K 9/107, A61K 31/245, A61K 9/00, A61K 47/20

(54) **EMULSION ZUR PHARMAZEUTISCHEN ANWENDUNG UND VERFAHREN ZU DEREN HERSTELLUNG**
EMULSION FOR PHARMACEUTICAL USE AND METHOD FOR THE PRODUCTION THEREOF
ÉMULSION POUR UNE APPLICATION PHARMACEUTIQUE ET PROCÉDÉ DE PRÉPARATION

(30) Priorität: 20.06.2012 WO PCT/DE2012/000638
(43) Veröffentlichungstag der Anmeldung: 29.04.2015
(73) Patentinhaber: Klose, Peter, 39291 Grabow (DE)
(72) Erfinder: Klose, Peter, 39291 Grabow (DE)
(74) Vertreter: Fischer, Volker
(86) Internationale Anmeldenummer: PCT/DE2013/000336
(87) Internationale Veröffentlichungsnummer: WO 2013/189477

(56) Entgegenhaltungen:
- AU-A1- 2005 200 643
- DE-C1- 4 499 552
- US-A1- 2007 207 965
- S Fehr ET AL: "Preparation of Nanoemulsions and Solid Lipid Nanoparticles by Extrusion", , 1 January 2010 (2010-01-01), XP055345694, Retrieved from the Internet: URL:http://www.pharmtech.tu-bs.de/files/bu njes/SFehr_Japan1003.pdf [retrieved on 2017-02-14]

## Beschreibung

Die Erfindung betrifft eine Emulsion zur pharmazeutischen Anwendung, die einen Wirkstoff enthält, wie in den Ansprüchen definiert. Die Erfindung betrifft des Weiteren allgemein ein Verfahren zur Herstellung einer einen Wirkstoff enthaltenden Emulsion, wobei die Art der Emulsion sowie deren Herstellung eine Wirkstoffverstärkung zur Folge hat.

Bisher werden Wirkstoffe dem Patienten direkt verabreicht oder in einem Adjuvans verteilt. Vor- und Nachteile bei der bisherigen Anwendung von Wirkstoffen werden im Folgenden anhand eines Beispiels mit dem Wirkstoff Procain erläutert. Procain ist ein Lokalanästhetikum vom Ester-Typ, das zuerst in der Zahnmedizin eingesetzt wurde.

Es ist hauptsächlich unter dem Markennamen Novocain® oder Novacaine bekannt. Erstmals synthetisiert wurde Procain im Jahre 1905 von dem deutschen Chemiker Alfred Einhorn, welcher dem Wirkstoff den Namen Novocain® zuordnete (von lat. novus "neu" und cain wie in Cocain). Novocain® war das erste synthetische Lokalanästhetikum.

Durch den Einsatz von Procain werden spannungsabhängige Natriumkanäle blockiert und somit der schnelle Natriumeinstrom (der für eine Depolarisation an der Zellmembran von Axonen zuständig ist) verhindert. Dadurch wird eine Reizweiterleitung unterbunden. In geringerem Maße werden auch andere lonenkanäle, wie zum Beispiel Kaliumkanäle, blockiert. Damit das Lokalanästhetikum am lonenkanal wirken kann, muss es in seiner unprotonierten Wirkform angreifen, was den Einsatz in entzündlichem Gewebe erschwert, da dort das pH-Gleichgewicht auf die Seite der protonierten Form verschoben ist.

Procain wird für die Lokalanästhesie nur noch selten verwendet, da hierfür wirksamere, weit tiefer eindringfähige Stoffe, wie etwa Lidocain, zur Verfügung stehen. Allerdings wird Procain weiterhin in der Neuraltherapie verwendet, einer Behandlungsform aus dem Bereich der Alternativmedizin.

Ein weiteres Anwendungsgebiet für das Procain besteht in der Krebstherapie. Relativ neu ist dabei die Entdeckung, dass Procain das Enzym DNA-Methylase hemmen kann. Dieser Umstand kann genutzt werden, um paragenetische Schäden der Genexpression rückgängig zu machen. Dies ist vor allem bei Schäden an sogenannten Tumorsuppressorgenen wie p53 interessant. Des Weiteren belegen Studien, wie von C. Fenoglio et al.: Anti-Cancer Drugs, November 2002, Volume 13, Issue 10, pp. 1043-1054, und A. Villar-Garea et al.: Procain is a DNA-demethylating Agent with Growth-inhibitory Effects in Human Cancer Cells, Cancer Research 2003, dass Procain bei isolierten menschlichen Brustkrebszellen das Wachstum verhindert. Procain wirkt darüber hinaus als Zellschutz der gesunden Zellen bei der Behandlung durch Chemotherapie.

Procain hat zudem eine gefäßerweiternde Wirkung, insbesondere in den Gefäßen von Herz, Lunge, Magen und Darm. Dadurch eignen sich procainhaltige Medikamente für die Behandlung von Herz-/Kreislauferkrankungen, wobei Procain Herzrhythmus stabilisierend wirkt und für eine Erweiterung der Herzkranzgefäße sorgt. Procain hemmt auch die körpereigene Produktion von Interleukin-6 und CRP als zwei wesentliche Risikofaktoren für Schlaganfall und Herzinfarkt.

Procain sorgt aufgrund seiner antioxidativen Eigenschaften darüber hinaus für eine Verzögerung der Zellalterung und die Stabilisierung der Zellmembran. Procain wirkt außerdem stimmungsaufhellend, besitzt dabei aber keine euphorisierende Wirkung wie Cocain und fällt daher nicht unter den Geltungsbereich der Betäubungsmittelgesetze und -verordnungen. Schließlich ist auch die entzündungshemmende Wirkung von Procain zu nennen.

Die Firma Schwarzhaupt (siehe www.schwarzhaupt-arznei.de/arznei/kh3.html) vertreibt in Kapselform und zur regelmäßigen Einnahme das procainhaltige Arzneimittel K.H.3® zur Aufrechterhaltung des körperlichen Wohlbefindens und der geistigen Leistungsfähigkeit, insbesondere bei älteren Menschen. Dabei bewirkt das Procain eine Aktivierung des Zellstoffwechsels und damit eine Verbesserung der Organ- und Drüsenfunktionen. Weitere Bestandteile im Arzneimittel sind dabei vorgesehen, um die Wirkungsstärke von Procain wesentlich zu steigern und die Wirkungsdauer zu verlängern.

Problematisch sind die Nebenwirkungen bei häufiger und regelmäßiger Einnahme von Procain. Zu diesen Nebenwirkungen von Procain zählen insbesondere:
- zentralnervöse Störungen, wie Unruhe, Delirium, tonisch-klonische Krämpfe,
- Ödeme,
- Urtikaria und lokale Allergien,
- Blutdruckabfall und Kreislaufreaktion,
- Brochospasmus sowie das Atemnotsyndrom.

Ein Weg, die Nebenwirkungen zu reduzieren, besteht darin, dass der Wirkstoff in eine Verabreichungsform überführt wird, bei der Wirkstoffe, die normalerweise erst in höher dosierter Form wirksam sind, auch bei geringerer Dosierung ihre Wirksamkeit behalten.

Die DE 44 99 552 C1 beschreibt Adjuvantien vom Typ der Öl-in-Wasser-Emulsionen für Antigene zur Immunisierung, wobei diese Adjuvantien die Funktion eines Depots haben, in das die Antigene eingebettet sind. Auf diese Weise soll erreicht werden, dass die Resorption parenteral verabreichter Antigene verlangsamt und dabei einen langer Antigenreiz ausgeübt wird. Dabei besteht die Öl-Phase aus Polydimethylsiloxanen und die wässrige Phase im Wesentlichen aus biokompatibler Salzlösung. Stabilisiert wird die Öl-Phase in der Wasser-Phase mittels eines Komplexemulgators, der einen HLB-Wert von 9 bis 16 aufweist. Der Komplexemulgator ist eine Kombination aus aliphatischen Alkoholen im Wesentlichen mit der Kettenlänge C10 bis C100 aus Sorbitol und/oder Glycerolfettsäureestern und aus Polysorbaten. Die biokompatible Salzlösung ist eine Phosphat gepufferte Natriumchloridlösung, die Chelatbildner enthält. Des Weiteren enthält die Kombination zusätzlich Dimethylsulfoxid, das sich entsprechend seinem Löslichkeitsverhalten in beiden Phasen verteilt.

Über Dimethylsulfoxid ist darüber hinaus durch M. Doege, Photoprotektive Mechanismen der Alge Euglena gracilis in Abhängigkeit vom Carotinoidgehalt: Untersuchungen zur nichtphotochemischen Löschung der Chlorophyll a - Fluireszenz, Dissertation, Martin-Luther-Universität Halle-Wittenberg, 1999, S. 18, bekannt, dass es auch in hohen Konzentrationen für biologische Systeme nicht toxisch wirkt, in Gewebe schnell aufgenommen und verteilt wird und sich dort unter anderem als ein idealer Hydroxylradikalfänger erweist. Dabei reagieren die Radikale mit DMSO unter Bildung eines stabilen Reaktionsproduktes - der Methylsulfinsäure - und eines Methylradikals: CH₃-SO-CH₃ + OH* → CH₃-SOOH + CH₃*

Durch G. Roewer, Institut für Anorganische Chemie, TU Bergakademie Freiberg, Vortrag vom 12.11.2004 in Lostau, wurde berichtet, dass sich Dimetikon, dessen Polymermoleküle die Dimethylsulfoxidmoleküle kugelförmig umschließen, als Radikalfänger für die durch den Zerfall von Dimethylsulfoxid entstehenden Methylradikale eignet, indem das Dimetikon die Methylradikale abfängt, deaktiviert und somit stabilisiert.

Prinzipiell ließen sich derartige Adjuvantien für die Verabreichung von Wirkstoffen, wie zum Beispiel Procain, anwenden, um eine Art Depotwirkung des zu verabreichenden Wirkstoffs zu erreichen und auf diese Weise die notwendige Menge zu reduzieren. Allerdings zeigten erste Versuche, dass durch eine einfache Kombination der beschriebenen Emulsion gemäß der DE 44 99 552 C1 und dem Wirkstoff eine Wirkstoffreduktion unter Beibehaltung beziehungsweise Steigerung der Wirkung nicht erreicht werden kann. Des Weiteren muss die erhaltene Emulsion gekühlt werden, um selbst über einen relativ kurzen Zeitraum stabil zu bleiben. Zudem zeigte sich, dass einige Emulsionsbestandteile bei deren Verabreichung zumindest in Teilbereichen der in der DE 44 99 552 C1 angegebenen Konzentrationen heftige Nebenwirkungen verursachten. So kam es bei der Injektion der Emulsion zu lokalen Reaktionen an der Injektionsstelle, wie Schwellungen, Rötungen und/oder Schmerzen. Auch führte die Anwendung vereinzelt zu Kreislaufreaktionen nach der Injektion, insbesondere zum sogenannten Flush.

Die AU 2005 200 643 A1 beschreibt eine Öl-in-Wasser-Emulsion, die für die Herstellung eines Medikaments gegen verschiedene Krankheiten, unter anderem gegen systemische Entzündung und Viruserkrankungen, verwendet werden kann. Diese Emulsion wird gemäß Beispiel 1 der Druckschrift D1 aus 0,01 bis 30 % Polydimethylsiloxanen, die einen Polymerisationsgrad von n = 20 bis 400 und eine kinematische Viskosität von 20 bis 1300 mm²/s aufweisen, 0,01 bis 15 % eines hydrophilen Emulgators, 45 bis 99 % einer biokompatiblen Salzlösung und 0,01 bis 10 % Demthylsulfoxid hergestellt. Es wird in Druckschrift D1 darauf verwiesen, dass diese Emulsion nach bekannten Vorschriften (known emulsion chemical norms) hergestellt wird.

Der Erfindung liegt die Aufgabe zugrunde, Wirkstoffe in eine solche Zusammensetzung und Form zu überführen, dass die Nebenwirkungen verringert werden und somit eine häufigere Anwendung ermöglicht wird. Des Weiteren soll dieses für die Verabreichung geeignete Wirkstoffgemisch über eine möglichst lange Zeit stabil bleiben.

Die Aufgabe der Erfindung wird gelöst durch eine Emulsion zur pharmazeutischen Anwendung, wie definiert in den Ansprüchen, die aus Emulsionskügelchen besteht, von denen mindestens 99 % jeweils einen Durchmesser aufweisen, der kleiner oder gleich einem Wert D₉₉ ≤ 3,5 µm ist, ermittelt mit der Methode der Laserdiffraktometrie, die die folgenden Komponenten enthält:
- einen Wirkstoff in einem Anteil von 0,001 % bis 50 %,
- 0,01 % bis 30 % Polydimethylsiloxanen, die einen Polymerisationsgrad von n = 20 bis 400 aufweisen,
- 0,01 % bis 15 % Komplexemulgator mit einem HLB-Wert von 9,16,
- 45 % bis 99 % biokompatible Salzlösung,
- 0,01 % bis 10 % Dimethylsulfoxid und
- 0 % bis 1 % Chelatbildner.

Erfindungsgemäß besteht die Emulsion dabei aus Emulsionskügelchen, von denen mindestens 99 % jeweils einen Durchmesser aufweisen, der kleiner oder gleich einem Wert D₉₉ ≤ 3,5 µm ist. Der D₉₉ -Wert wird mit der Methode der Laserdiffraktometrie ermittelt. Eine solche Bestimmung ist zum Beispiel mit einem Gerät vom Typ LS 230 Particle Sizer, Small Volume Modul Beckman-Coulter-GmbH, Krefeld-Deutschland, möglich, welches die Laserdiffraktometrie mit der Methode der PIDS-Technologie (PIDS= Polarization Intensity Differential Scattering Technology) kombiniert.

Vorzugsweise weisen die verwendeten Polydimethylsiloxane eine kinematische Viskosität von 4 bis 250 mm²/s bei 20 °C auf, gemessen mit der Methode der Kapillarviskosimetrie nach den Anforderungen des Europäischen Arzneibuchs, Grundwerk 2008, Ph.Eur.6.0/2.2.9 Kapillarviskosimetrie, wobei zum Beispiel ein Ubbelohde-Kapillarviskosimeter verwendet werden kann. Vorteilhaft ist aber auch der Einsatz von Polydimethylsiloxan mit einer ebenfalls mit der Methode der Kapillarviskosimetrie nach den Anforderungen des Europäischen Arzneibuchs gemessenen kinematischen Viskosität von 350 mm²/s bei 25 °C, wie es zum Beispiel nominal unter der Bezeichnung Dimeticon 350 bekannt ist.

Gemäß der Konzeption der Erfindung wird der Wirkstoff, zum Beispiel Procain, in ein Substanzgemisch aus Ölen, Emulgatoren und anderen Zusätzen gebracht, um es in diesem Gemisch dem Patienten verabreichen zu können. Es konnte überraschenderweise eine Wirkverstärkung beobachtet werden, die viel größer war, als es allein durch die infolge der Mikroemulsion angestrebte bessere Präsentation des Wirkstoffs im Gewebe erwartet werden konnte. Offensichtlich kommt dabei ein Synergieeffekt mit dem Radikalfänger Dimethylsulfoxid zum Tragen, wobei das Dimethylsulfoxid, wie bereits erwähnt, innerhalb der Emulsionskügelchen von den Polydimethylsiloxanketten umschlossen wird. Aufgrund der Mikroemulsion erfolgt daher eine bessere Verteilung sowohl des Wirkstoffes als auch des Dimethylsulfoxids im Körper, wobei die relativ schwachen van der Waals-Kräfte, die das Dimethylsulfoxid und den Wirkstoff an die Polydimethylsiloxanketten binden, innerhalb des Körpers aufgebrochen werden können. Wenn der Wirkstoff im Körper abgegeben ist, kann die Emulsion, die Polydimethylsiloxanketten und Dimethylsulfoxid enthält, aufgrund der genannten Eigenschaften den Wirkstoff weiter unterstützen, indem sie Sauerstoffradikale bindet und reduziert, wenn Dimethylsulfoxid freigesetzt wird.

Aufgrund der Mengenreduktion, die infolge der Wirkverstärkung ermöglicht wird, besteht keine Überdosierungsgefahr mehr. Weiterhin kann so auch eine Reduktion der überschießenden Wirkungen und Nebenwirkungen erreicht werden. Das heißt, dass auch der häufigere Einsatz des Wirkstoffes dadurch möglich ist. Dabei ist die erfindungsgemäße Emulsion geeignet, als Zäpfchen, in einer Spritze und in Form von Kapseln zugesetzt zu werden.

In einer bevorzugten Ausführungsform der Erfindung bestehen die Komplexemulgatoren aus aliphatischen Alkoholen der Kettenlängen C10 bis C100 aus Sorbitol- und/oder Glycerolfettsäureester und aus Polysorbaten. Dabei beträgt das Massenverhältnis von aliphatischen Alkoholen, Sorbitol- und/oder Glycerolfettsäureester und Polysorbaten vorzugsweise 0,5 bis 3 zu 0,2 bis 4 zu 1 bis 5.

Bevorzugt wird der Anteil der Komplexemulgatoren in der Emulsion bei höchstens 3 % gehalten. In höherer Konzentration sorgt gerade dieser Teil immer wieder für lokale Reaktionen an der Injektionsstelle, insbesondere bei der Anwendung von Cetylstearylalkohol (Trivialname für ein Gemisch aus Hexadecanol und Octadecanol). Durch die Begrenzung des Anteils dieses Komplexemulgators auf 3 % konnte die Verträglichkeit erheblich verbessert werden, ohne dass die Funktion des Komplexemulgators als Stabilisator des Öl-Wasser-Gemisches negativ beeinträchtigt wurde.

Als Chelatbildner sind bevorzugt Salze der Ethylendiamintetraessigsäure (EDTA) enthalten. Zweckmäßig ist es, mehrwertige, wasserlösliche Alkohole wie Glycerol zuzusetzen, die dann vorzugsweise in einer Konzentration von 0,01 % bis 10 % im Wirkstoff enthaltenden Gemisch vorliegen.
Außer mit Procain können auch mit anderen Wirkstoffen erfindungsgemäße Emulsionen gebildet werden. Das betrifft Wirkstoffe der Wirkstoffgruppen Acidosetherapeutika, Analeptika/Antihypoxämika, Analgetika/Antirheumatika, Antiallergika, Antianämika, Antiarrhythmika, Antibiotika/Antiinfektiva, Antidementiva, Antidiabetika, Antidota, Antiepileptika, Antihämorrhagika, Antihypertonika, Antihypoglykämika, Antihypotonika, Antikoagulantia, Antimykotika, Antiphlogistika, Antitussiva/Expektorantia, Arteriosklerosemittel, Balneotherapeutika und Mittel zur Wärmetherapie, Betarezeptoren-, Calciumkanalblocker und Hemmstoffe des Renin-Angiotensin-Aldosteron-Systems, Broncholytika/Antiasthmatika, Cholagoga und Gallenwegstherapeutika, Cholinergika, Corticoide, Dermatika, Diuretika, durchblutungsfördernde Mittel, Enzyminhibitoren, Präparate bei Enzymmangel und Transportproteine, Fibrinolytika, Geriatrika, Gichtmittel, Grippemittel und Mittel gegen Erkältungskrankheiten, Gynäkologika, Hämorrhoidenmittel, Hepatika, Hypnotika/Sedativa, Hypophysen-, Hypothalamushormone, andere regulatorische Peptide und ihre Hemmstoffe, Immunmodulatoren, Kardiaka, Karies-, Parodontosemittel und andere Dentalpräparate, Koronarmittel, Lipidsenker, Lokalanästhetika/Neurahtherapeutika, Magen-Darm-Mittel, Migränemittel, Mineralstoffpräparate, Mund- und Rachentherapeutika, Muskelrelaxanzien und -Reversoren, Ophthalmika, Osteoporosemittel/Calcium-/Knochenstoffwechselregulatoren, Otologika, Parkinsonmittel und andere Mittel gegen extrapyramidale Störungen, Psychopharmaka, Rhinologika/Sinusitismittel, Roborantia/Tonika, Schilddrüsentherapeutika, Sera, Immunglobuline und Impfstoffe, Sexualhormone und ihre Hemmstoffe, Spasmolytika/Anticholinergika, Thrombozytenaggregationshemmer, Tuberkulosemittel, Umstimmungsmittel, Urologika, Venentherapeutika, Vitamine, Wund- und Narbenbehandlungsmittel, Zytostatika, andere antineoplastische Mittel und Protektiva und registrierte Homöopathika/Präparateserien.

Ein weiterer Aspekt der Erfindung betrifft ein Verfahren zur Herstellung einer erfindungsgemäßen Emulsion. Hierbei wird nicht, wie sonst bei Grippeimpfungen, der Wirkstoff zur fertigen Emulsion dazugegeben, sondern die Emulsion zusammen mit dem Wirkstoff angefertigt. Es wird nicht einfach eine Emulsion hergestellt, sondern es wird unter besonderen Temperaturbedingungen ultraschnell die Emulsion erzeugt, da nur auf diese Weise gewährleistet werden kann, dass eine dauerhafte Stabilisierung durch Anbindung des Wirkstoffs, zum Beispiel Procain, auf die Emulsionskügelchen des neuen Medikaments beziehungsweise pharmazeutischen Mittels erreicht werden kann.

Das erfindungsgemäße Verfahren umfasst im Allgemeinen die aufeinanderfolgenden Schritte:
a) getrenntes Einwiegen des Wirkstoffes und des Chelatbildners;
b) Einwiegen und Schmelzen eines Gemisches aus den gewünschten Anteilen der Polydimethylsiloxane, des Dimethylsulfoxids und der Komplexemulgatorkomponenten in einem ersten Gefäß bei 55 °C zur Cremeschmelze;
c) Einwiegen, Herstellen und Erwärmen einer Kochsalzlösung und anschließendes Abkühlen der Kochsalzlösung auf eine Temperatur in einem Temperaturbereich von 45 bis 55 °C in einem zweiten Gefäß;
d) Zugabe des Wirkstoffs oder einer wasserlöslichen Verbindung des Wirkstoffs sowie Zugabe des Chelatbildners in das zweite Gefäß, wobei beide Komponenten in Lösung gebracht werden;
e) Vereinigen der Inhalte des ersten Gefäßes und des zweiten Gefäßes;
f) kontinuierliches Homogenisieren der vereinigten Lösungen aus dem ersten Gefäß und dem zweiten Gefäß für mindestens fünf Minuten in einem Rotor-Stator-Homogenisator in einem Rotorgeschwindigkeitsbereich von mindestens 17000 Umdrehungen pro Minute und anschließendes Absetzenlassen einer durch das Homogenisieren gebildeten Schaumschicht;
g) Abfüllen des gesamten Gemisches der vereinigten Lösungen in Abfüllgefäße, die bei einer Temperatur im Bereich zwischen 110 und 125 °C autoklaviert werden und
h) Rehomogenisierung des Gemisches durch Kaltschütteln der Abfüllgefäße in einer Schüttelmaschine.

Grundsätzlich gilt, dass eine höhere Zahl an Umdrehungen eine größere Stabilität der Emulsion zur Folge hat. Die neue Emulsion muss bei Herstellung zwingend wenigstens 5 Minuten lang bei mindestens 17000 U/min emulgiert werden. Ebenso müssen die angegebenen Temperaturbereiche exakt eingehalten werden. Erst durch die Ultrahomogenisierung werden kleinste Emulsionströpfchen im Nanobereich hergestellt. Erst dadurch kann der Wirkstoff zusammen mit dem Radikalfänger Dimethylsulfoxid stabil in die Ketten der Polydimethylsiloxane eingebettet werden. Überraschenderweise kann dadurch eine Stabilität, auch bei Zimmertemperatur, über wesentlich längere Zeiträume als bei bisherigen Emulsionen, das heißt für mehrere Jahre, erreicht werden.

Mit dem Verfahren werden die verschiedenen, zum Teil bereits genannten Vorteile erreicht, indem der Wirkstoff durch eine Emulgierung im Nanobereich verteilt und verfügbarer wird. Es gelingt, eine Emulsion herzustellen, welche überraschenderweise so starke van der Waals-Kräfte bildet, dass der Wirkstoff auf die kleinen Emulsionskügelchen (vorzugsweise circa drei Mikrometer im Durchmesser) angedockt wird, ohne ihn dabei zu verändern. Der Wirkstoff wird jetzt mit einer wesentlich größeren Oberfläche im Körper präsentiert. Die Bioverfügbarkeit und die Durchdringung wird verbessert. Infolgedessen ist für die gleiche Wirkung weniger Menge notwendig. Durch die Verknüpfung mit der Emulsion entsteht ein Depot-Effekt im Körper und somit eine Wirkungsverlängerung. So werden die Nebenwirkungen verringert, die Wirksamkeit gesteigert und die Toxizität herabgesetzt. Durch dieses Verfahren besteht somit die Möglichkeit, nahezu aus jedem Medikament ein neues, besser verträgliches Medikament zu kreieren. Zudem ist die erfindungsgemäße Emulsion mit diesem Verfahren vorteilhafterweise auch im großindustriellen Maßstab erzeugbar.

In einer bevorzugten Ausführungsform des erfindungsgemäßen Verfahrens umfasst das Verfahren die folgenden Verfahrensschritte:
a) getrenntes Einwiegen des Wirkstoffes und von CaNa₂EDTA als Chelatbildner;
b) Einwiegen und Schmelzen eines Gemisches aus Dimeticon, Dimethylsulfoxid, Sorbitantrioleat, Cetylstearylalkohol, Polysorbat 80, Glycerol in einem ersten Gefäß bei 55 °C zur Cremeschmelze;
c) Herstellen und Erwärmen einer Kochsalzlösung und anschließendes Abkühlen der Kochsalzlösung auf eine Temperatur von 50 °C in einem zweiten Gefäß;
d) Zugabe von Procaindihydrochlorid sowie Zugabe von CaNa₂EDTA als Chelatbildner in das zweite Gefäß, wobei beide Komponenten in Lösung gebracht werden;
e) Vereinigen der Inhalte des ersten Gefäßes und des zweiten Gefäßes;
f) kontinuierliches Homogenisieren der vereinigten Lösungen aus dem ersten Gefäß und dem zweiten Gefäß für mindestens fünf Minuten in einem Rotor-Stator-Homogenisator in einer Rotorgeschwindigkeit von 17000 Umdrehungen pro Minute und anschließendes Absetzenlassen einer durch das Homogenisieren gebildeten Schaumschicht;
g) Abfüllen des gesamten Gemisches der vereinigten Lösungen in Rollrandflaschen, die bei einer Temperatur von 121 °C autoklaviert werden; und
h) Rehomogenisierung des Gemisches durch Kaltschütteln der Rollrandflaschen in einer Schüttelmaschine.

Schließlich besteht ein weiterer Aspekt der Erfindung in einer pharmazeutischen Zusammensetzung, die eine erfindungsgemäße Emulsion enthält und die sich zur oralen, rektalen oder vaginalen Verabreichung eignet. Diese Zusammensetzung eignet sich vorzugsweise zur Herstellung eines neuen pharmazeutischen Mittels in Form einer Tablette, einer Kapsel oder eines Zäpfchens.

Alternativ ist eine pharmazeutische Zusammensetzung vorgesehen, die eine erfindungsgemäße Emulsion enthält und die sich zur topischen Verabreichung eignet. Eine solche pharmazeutische Zusammensetzung eignet sich vorteilhafterweise zur Herstellung eines pharmazeutischen Mittels in Form einer Salbe.

Eine Anwendung der erfindungsgemäßen Emulsion ist sowohl in der Human- als auch in der Tiermedizin vorgesehen.

Weitere Einzelheiten, Merkmale und Vorteile der Erfindung ergeben sich aus der nachfolgenden Beschreibung von Ausführungsbeispielen.

Eine Emulsion mit dem Wirkstoff Procain dient dabei als Ausführungsbeispiel.

Im Folgenden wird ein Verfahren zur Herstellung einer erfindungsgemäßen Emulsion am Beispiel einer Emulsion mit dem Wirkstoff Procain beschrieben:
Zunächst werden die Gefäße zur Herstellung mit 70%igem Ethanol desinfiziert.

Dann erfolgen Einzeleinwaagen des Wirkstoffes sowie des Chelatbildners. Als Wirkstoff ist Procain vorgesehen.

Einzeleinwaagen: Procainhydrochlorid wird mittels eines Kartenblattes auf einer Feinwaage eingewogen. Calciumtriplex ® (CaNa₂EDTA) wird als Chelatbildner auf einem Kartenblatt auf der Analysenwaage eingewogen.
Schritt 1: In einem ersten Gefäß Nr. 1 (Becherglas 500 ml und Glasstab) werden eingewogen:
   Dimeticon, Dimethylsulfoxid, Sorbitantrioleat, Cetylstearylalkohol, Polysorbat 80, Glycerol.
   Das hergestellte Gemisch wird auf dem Wasserbad bei 55 °C zur Cremeschmelze geschmolzen, wobei keine Lanetteteilchen sichtbar sein dürfen.
Schritt 2: In das zweite Gefäß Nr. 2, einem 1 Liter-Becherglas, wird eine Kochsalzlösung auf der Feinwaage eingewogen. Das Ganze wird auf zirka 65 °C durch Indirekt-Heizung erwärmt. Danach wird die Lösung auf zirka 50 °C abgekühlt. Anschließend wird dieser Lösung Calciumtriplex sowie Procaindihydrochlorid zugegeben und alles in Lösung gebracht.
Schritt 3: Der Inhalt aus dem zweiten Gefäß Nr. 2 wird in das erste Gefäß Nr. 1 gegossen und umgekehrt. Durch mehrmaliges Spülen werden die Reste aus dem zweiten Gefäß Nr. 2 vollständig in das erste Gefäß Nr. 1 übernommen.
Schritt 4: Die Lösung im ersten Gefäß Nr. 1 wird fünf Minuten kontinuierlich mit dem Homogenisator Gastro 2000® bei einer Umdrehungszahl von 17000 U/min, was der Stufe 2 entspricht, homogenisiert.
Schritt 5: Die sich durch das Homogenisieren entwickelte Schaumschicht an der Oberfläche des Gefäßes wird zirka zwei Stunden abgedeckt stehengelassen, bis sich die Schaumschicht abgesetzt hat.
Schritt 6: Der Ansatz wird mit Hilfe eines kleineren Becherglases (zum besseren Abfüllen) in vorher mit 70%igen Ethanol desinfizierten Rollrandflaschen konfektioniert.
Schritt 7: Die Flaschen werden mit einem Injektionsstopfen aus Chlorbutylkautschuk verschlossen. Die Alukappen werden darauf gesetzt und mit einer Handbördelzange zugebördelt.
Schritt 8: Die Rollrandflaschen werden im Autoklaven bei 121 °C über 15 Minuten autoklaviert, wobei bei 70 °C das Ventil vom Autoklaven zugedreht wird. Nach Ende der Autoklavierung und vorsichtigem Entlüften der Autoklavenkammer bei 90 °C werden die Rollrandflaschen - ebenfalls vorsichtig - mit der Tiegelzange entnommen und in die Schüttelauflage der Schüttelmaschine gelegt.
Schritt 9: Die Rehomogenisierung erfolgt durch Kaltschütteln der Rollrandflaschen mittels der Schüttelmaschine bei 180 Mot/min für 6 Stunden. Danach werden die Rollrandflaschen bei Zimmertemperatur aufbewahrt und nach 24 bis 36 Stunden erneut für zirka 1 Stunde bei einer gleichbleibenden Geschwindigkeit von 180 Mot/min geschüttelt. Schließlich werden die fertigen Flaschen mit entsprechend bedruckten Haftetiketten versehen.

### Wirkungssteigerung am Beispiel von Procain:

Die Wirkung von 5 ml gespritztem Injektionsmedikament-Neu subcutan aus einer Mischung in Form einer 0,1 %igen Emulsionslösung von 1 %igem Procain ist überraschenderweise gleich der Wirkung von 0,2 ml 1 %igem Procain. Demnach führt die Anwendung einer 200fache Verdünnung des Procains zum gleichen Effekt, zum Beispiel bei der Linderung von Schmerzen.

Die Emulsion kann in Form einer Injektion, einer Salbe oder einer Kapsel verabreicht werden.

### Tonicum Zäpfchen mit Aesculus D6 als Wirkstoff:

### Rp. 10 g Zäpfchen enthalten:

| | |
|---|---|
| Aesculus D6 | 0,50 g |
| Dimethylsulfoxid | 0,83 g |
| Dimeticon 350 | 0,82 g |
| Oleum Cacao (Kakaobutter) | 4,93 g |
| Lanette® O (50: 50 Cetylalkohol und Stearylalkohol) | 1,15 g |
| Polysorbat 80 | 0,31 g |
| Glycerol | 0,98 g |
| Isotonische Natriumchloridlösung ad | 10,00 g. |

### Salbe zur topischen Behandlung mit Dimethylsulfoxid als Wirkstoff

### Rp 10 ml Salbe enthalten:

| | |
|---|---|
| Dimethylsulfoxid | 0,74 g |
| Dimeticon 350 | 0,32 g |
| Unguentum emulsificans aquosum (wasserhaltige hydrophile Salbe) DAB | 5,90 g |
| Lanette® N (emulgierender Cetylstearylalkohol) | 0,53 g |
| Polysorbat 80 | 0,10 g |
| Glycerol | 0,64 g |
| isotonische Natriumchloridlösung ad | 10,00 g. |

In einem weiteren Beispiel liegt die pharmazeutische Zusammensetzung mit einer erfindungsgemäßen Emulsion in einem pharmazeutischen Mittel in fester Form vor, das heißt in einer Tablette, einer Kapsel oder einem Zäpfchen. Dabei enthält die Emulsion, bezogen auf 10 g der pharmazeutischen Zusammensetzung, 0,01 g bis 0,5 g Allicin.

### Knoblauch Zäpfchen:

### Rp. 10 g Zäpfchen enthalten:

| | |
|---|---|
| Allicin | 0,15 g |
| Dimethylsulfoxid | 0,83 g |
| Dimeticon 350 | 0,82 g |
| Oleum Cacao (Kakaobutter) | 4,93 g |
| Lanette® O (50 : 50 Cetylalkohol und Stearylalkohol) | 1,15 g |
| Polysorbat 80 | 0,31 g |
| Glycerol | 0,98 g |
| Isotonische Natriumchloridlösung ad | 10,00 g. |

## Patentansprüche

1. Emulsion zur pharmazeutischen Anwendung, wobei die Emulsion aus Emulsionskügelchen besteht, von denen mindestens 99 % jeweils einen Durchmesser aufweisen, der kleiner oder gleich einem Wert D₉₉ ≤ 3,5 µm ist, ermittelt mit der Methode der Laserdiffraktometrie, enthaltend
- einen Wirkstoff in einem Anteil von 0,001 % bis 50 %,
- 0,01 % bis 30 % Polydimethylsiloxanen, die einen Polymerisationsgrad von n = 20 bis 400 aufweisen,
- 0,01 % bis 15 % Komplexemulgator mit einem HLB-Wert von 9,16,
- 45 % bis 99 % biokompatible Salzlösung,
- 0,01 % bis 10 % Dimethylsulfoxid und
- 0 % bis 1 % Chelatbildner,
wobei der Wirkstoff ausgewählt wird aus den Wirkstoffen/Wirkstoffgruppen Procain, Acidotherapeutika, Analeptika/Antihypoxämika, Analgetika/Antirheumatika, Antiallergika, Antianämika, Antiarrhythmika, Antibiotika/Antiinfektiva, Antidementiva, Antidiabetika, Antidota, Antiepileptika, Antihämorrhagika, Antihypertonika, Antihypoglykämika, Antihypotonika, Antikoagulantia, Antimykotika, Antiphlogistika, Antitussiva/Expektorantia, Arteriosklerosemittel, Balneotherapeutika und Mittel zur Wärmetherapie, Betarezeptoren-, Calciumkanalblocker und Hemmstoffe des Renin-Angiotensin-Aldosteron-Systems, Broncholytika/Antiasthmatika, Cholagoga und Gallenwegstherapeutika, Colinergika, Corticoide, Dermatika, Diuretika, durchblutungsfördernde Mittel, Enzyminhibitoren, Präparate bei Enzymmangel und Transportproteine, Fibrinolytika, Geriatrika, Gichtmittel, Grippemittel und Mittel gegen Erkältungskrankheiten, Gynäkologika, Hämorrhoidenmittel, Hepatika, Hypnotika/Sedativa, Hypophysen-, Hypothalamushormone, andere regulatorische Peptide und ihre Hemmstoffe, Immunmodulatoren, Kardiaka, Karies-, Paradontosemittel und andere Dentalpräparate, Koronarmittel, Lipidsenker, Lokalanästhetika/Neuraltherapeutika, Magen-Darm-Mittel, Migränemittel, Mineralstoffpräparate, Mund- und Rachentherapeutika, Muskelrelaxanzien und - Reversoren, Ophthalmika, Osteoporosemittel/Calcium-/Knochenwechselregulatoren, Otologika, Parkinsonmittel und andere Mittel gegen extrapyramidale Störungen, Psychopharmaka, Rhinologika/Sinusitismittel, Roborantia/Tonika, Schilddrüsentherapeutika, Sera, Immunglobine und Impfstoffe, Sexualhormone und ihre Hemmstoffe, Spasmolytika/Anticholinergika, Thrombozytenaggregationshemmer, Tuberkulosemittel, Umstimmungsmittel, Urologika, Venentherapeutika, Vitamine, Wund- und Narbenbehandlungsmittel, Zytostatika, andere antineoplastische Mittel und Protektiva und registrierte Homöopathika/Präparateserien.

2. Emulsion nach Anspruch 1,
**dadurch gekennzeichnet,**
**dass** die Komplexemulgatoren aus aliphatischen Alkoholen der Kettenlängen C10 bis C100, aus Sorbitol- und/oder Glycerolfettsäureester und aus Polysorbaten bestehen.

3. Emulsion nach einem der Ansprüche 1 und 2,
**dadurch gekennzeichnet,**
**dass** zusätzlich mehrwertige, wasserlösliche Alkohole, bevorzugt Glycerol, in einer Konzentration von 0,01 % bis 10 % enthalten sind.

4. Emulsion nach einem der Ansprüche 1 bis 3,
**dadurch gekennzeichnet,**
**dass** der Anteil der Komplexemulgatoren in der Emulsion nicht größer als 3 % ist.

5. Verfahren zur Herstellung einer einen Wirkstoff enthaltenden Emulsion nach einem der Ansprüche 1 bis 4, umfassend die Verfahrensschritte:
a) getrenntes Einwiegen des Wirkstoffes und des Chelatbildners;
b) Einwiegen und Schmelzen eines Gemisches aus den gewünschten Anteilen der Polydimethylsiloxane, des Dimethylsulfoxids und der Komplexemulgatorkomponenten in einem ersten Gefäß bei 55 °C zur Cremeschmelze;
c) Einwiegen, Herstellen und Erwärmen einer Kochsalzlösung und anschließendes Abkühlen der Kochsalzlösung auf eine Temperatur in einem Temperaturbereich von 45 °C bis 55 °C in einem zweiten Gefäß;
d) Zugabe des Wirkstoffs oder einer wasserlöslichen Verbindung des Wirkstoffs sowie Zugabe des Chelatbildners in das zweite Gefäß, wobei beide Komponenten in Lösung gebracht werden;
e) Vereinigen der Inhalte des ersten Gefäßes und des zweiten Gefäßes;
f) kontinuierliches Homogenisieren der vereinigten Lösungen aus dem ersten Gefäß und dem zweiten Gefäß für mindestens fünf Minuten in einem Rotor-Stator-Homogenisator in einem Rotorgeschwindigkeitsbereich von mindestens 17000 Umdrehungen pro Minute und anschließendes Absetzenlassen einer durch das Homogenisieren gebildeten Schaumschicht;
g) Abfüllen des gesamten Gemisches in Abfüllgefäße, die bei einer Temperatur im Bereich zwischen 110 und 125 °C autoklaviert werden und
h) Rehomogenisierung des Gemisches durch Kaltschütteln der Abfüllgefäße in einer Schüttelmaschine.

6. Verfahren nach Anspruch 5,
**gekennzeichnet durch** die Verfahrensschritte:
a) getrenntes Einwiegen des Wirkstoffes und von CaNa₂EDTA als Chelatbildner;
b) Einwiegen und Schmelzen eines Gemisches aus Dimeticon, Dimethylsulfoxid, Sorbitantrioleat, Cetylstearylalkohol, Polysorbat 80, Glycerol in einem ersten Gefäß bei 55 °C zur Cremeschmelze;
c) Herstellen und Erwärmen einer Kochsalzlösung und anschließendes Abkühlen der Kochsalzlösung auf eine Temperatur von 50 °C in einem zweiten Gefäß;
d) Zugabe von Procaindihydrochlorid sowie Zugabe von CaNa₂EDTA als Chelatbildner in das zweite Gefäß, wobei beide Komponenten in Lösung gebracht werden;
e) Vereinigen der Inhalte des ersten Gefäßes und des zweiten Gefäßes;
f) kontinuierliches Homogenisieren der vereinigten Lösungen aus dem ersten Gefäß und dem zweiten Gefäß für mindestens fünf Minuten in einem Rotor-Stator-Homogenisator in einer Rotorgeschwindigkeit von 17000 Umdrehungen pro Minute und anschließendes Absetzenlassen einer durch das Homogenisieren gebildeten Schaumschicht;
g) Abfüllen des gesamten Gemisches in Rollrandflaschen, die bei einer Temperatur von 121 °C autoklaviert werden und
h) Rehomogenisierung des Gemisches durch Kaltschütteln der Rollrandflaschen in einer Schüttelmaschine.

7. Pharmazeutische Zusammensetzung, die eine Emulsion nach einem der Ansprüche 1 bis 4 enthält und die sich zur oralen, rektalen oder vaginalen Verabreichung eignet.

8. Pharmazeutische Zusammensetzung nach Anspruch 7,
die sich zur Herstellung eines pharmazeutischen Mittels in Form einer Tablette, einer Kapsel oder eines Zäpfchens eignet.

9. Pharmazeutische Zusammensetzung nach Anspruch 8,
**dadurch gekennzeichnet,**
**dass** 10 g eines Zäpchens enthalten:
| | |
|---|---|
| Aesculus D6 | 0,50 g |
| Dimethylsulfoxid | 0,83 g |
| Dimeticon 350 | 0,82 g |
| Oleum Cacao (Kakaobutter) | 4,93 g |
| 50 : 50 Cetylalkohol und Stearylalkohol | 1,15 g |
| Polysorbat 80 | 0,31 g |
| Glycerol | 0,98 g |
| Isotonische Natriumchloridlösung ad | 10,00 g |

10. Pharmazeutische Zusammensetzung nach Anspruch 8,
**dadurch gekennzeichnet,**
**dass** die Emulsion, bezogen auf 10 g der pharmazeutischen Zusammensetzung, 0,01 g bis 0,5 g Allicin enthält.

11. Pharmazeutische Zusammensetzung nach Anspruch 10,
**dadurch gekennzeichnet,**
**dass** 10 g eines Zäpchens enthalten:
| | |
|---|---|
| Allicin | 0,15 g |
| Dimethylsulfoxid | 0,83 g |
| Dimeticon 350 | 0,82 g |
| Oleum Cacao (Kakaobutter) | 4,93 g |
| 50 : 50 Cetylalkohol und Stearylalkohol | 1,15 g |
| Polysorbat 80 | 0,31 g |
| Glycerol | 0,98 g |
| Isotonische Natriumchloridlösung ad | 10,00 g |

12. Pharmazeutische Zusammensetzung, die eine Emulsion nach einem der Ansprüche 1 bis 4 enthält und die sich zur topischen Verabreichung eignet.

13. Pharmazeutische Zusammensetzung nach Anspruch 12, die sich zur Herstellung eines pharmazeutischen Mittels in Form einer Salbe eignet.

## Claims

1. Emulsion for pharmaceutical use, wherein the emulsion consists of emulsion beads, at least 99% of which each have a diameter that is less than or equal to a D₉₉ value of ≤ 3.5 µm, determined by the laser diffractometry method, comprising
- an active agent in a proportion of 0.001% to 50%,
- 0.01% to 30% of polydimethylsiloxanes which have a degree of polymerization of n = 20 to 400,
- 0.01% to 15% of complex emulsifier with an HLB value of 9.16,
- 45% to 99% of biocompatible salt solution,
- 0.01% to 10% of dimethyl sulfoxide and
- 0 to 1% of chelating agents,
wherein the active agent is selected from the active agents/groups of active agents procaine, acidosis therapeutics, analeptics/antihypoxics, analgesixs/antirheumatics, antiallergenics, antianaemics, antiarrhythmics, antibiotics/antiinfectives, antidementia drugs, antidiabetics, antidotes, antiepileptics, antihaemorrhagics, antihypertonics, antihypoglycaemics, antihypotonics, anticoagulants, antimycotics, antiphlogistics, antitussives/expectorants, atherosclerosis compositions, balneotherapeutic agents and agents for heat therapy, beta receptor blockers, calcium channel blockers and renin-angiotensin-aldosterone system inhibitors, broncholytics/antiasthmatics, cholagogues and bile duct therapeutics, cholinergics, corticoids, dermatics, diuretics, blood flow-stimulating agents, enzyme inhibitors, preparations for enzyme deficiency and transport proteins, fibrinolytics, geriatric drugs, antigout agents, flu drugs and compositions for cold-related illnesses, gynaecological drugs, haemorrhoid drugs, hepatic drugs, hypnotics/sedatives, hypophysis hormones, hypothalamus hormones, other regulatory peptides and inhibitors thereof, immunomodulatory agents, cardiac drugs, caries compositions, paradontosis compositions and other dental preparations, coronary compositions, lipid-lowering drugs, local anaesthetics/neurotherapeutics, gastrointestinal compositions, migraine compositions, mineral preparations, oral-pharyngeal therapeutics, muscle relaxants and reversal agents, ophthalmic agents, osteoporosis agents/calcium/bone exchange regulators, otologics, Parkinson's drugs and other agents for combating extrapyramidal disorders, psychopharmaceuticals, rhinological/sinusitis drugs, roborants/tonics, thyroid therapeutics, sera, immunoglobulins and vaccines, sex hormones and inhibitors thereof, spasmolytics/anticholinergics, platelet aggregation inhibitors, tuberculosis drugs, stimulants, urologicals, vein therapeutics, vitamins, wound and scar treatment agents, cytostatics, other antineoplastics and protectants and registered homeopathics/product ranges.

2. Emulsion according to Claim 1,
**characterized in that**
the complex emulsifiers consist of aliphatic alcohols with a chain length of C10 to C100, of sorbitol and/or glycerol fatty acid esters and of polysorbates.

3. Emulsion according to one of Claims 1 and 2,
**characterized in that**
additional polyhydric water-soluble alcohols, preferably glycerol, are present in a concentration of 0.01% to 10%.

4. Emulsion according to one of Claims 1 to 3,
**characterized in that**
the proportion of complex emulsifiers in the emulsion is not greater than 3%.

5. Method for preparing an emulsion comprising an active agent according to one of Claims 1 to 4, comprising the method steps:
a) separately weighing in the active agent and the chelating agent;
b) weighing in and melting a mixture of the desired proportions of polydimethylsiloxane, dimethyl sulfoxide and complex emulsifier components in a first vessel at 55°C to give a cream melt;
c) weighing in, preparing and heating a sodium chloride solution and subsequently cooling the sodium chloride solution to a temperature in the temperature range from 45°C to 55°C in a second vessel;
d) adding the active agent or a water-soluble compound of the active agent and also adding the chelating agent in the second vessel, both components being placed in solution;
e) combining the contents of the first vessel and the second vessel;
f) continuously homogenizing the combined solutions from the first vessel and the second vessel for at least five minutes in a rotor-stator homogenizer in a rotor speed range of at least 17 000 revolutions per minute, and subsequently allowing a foam layer, formed by the homogenization, to settle;
g) decanting the entire mixture into decanting vessels which are autoclaved at a temperature in a range between 110 and 125°C, and
h) re-homogenizing the mixture by cold agitation of the decanting vessels in an agitator.

6. Method according to Claim 5,
**characterized by** the method steps:
a) separately weighing in the active agent and CaNa₂EDTA as chelating agent;
b) weighing in and melting a mixture of dimethicone, dimethyl sulfoxide, sorbitan trioleate, cetylstearyl alcohol, polysorbate 80 and glycerol in a first vessel at 55°C to give a cream melt;
c) preparing and heating a sodium chloride solution and subsequently cooling the sodium chloride solution to a temperature of 50°C in a second vessel;
d) adding procaine dihydrochloride and also adding CaNa₂EDTA as chelating agent in the second vessel, both components being placed in solution;
e) combining the contents of the first vessel and the second vessel;
f) continuously homogenizing the combined solutions from the first vessel and the second vessel for at least five minutes in a rotor-stator homogenizer at a rotor speed of at least 17 000 revolutions per minute, and subsequently allowing a foam layer, formed by the homogenization, to settle;
g) decanting the entire mixture into beaded-edge bottles which are autoclaved at a temperature of 121°C, and
h) re-homogenizing the mixture by cold agitation of the beaded-edge bottles in an agitator.

7. Pharmaceutical composition comprising an emulsion according to one of Claims 1 to 4 and which is suitable for oral, rectal or vaginal administration.

8. Pharmaceutical composition according to Claim 7, which is suitable for preparation of a pharmaceutical product in the form of a tablet, a capsule or a suppository.

9. Pharmaceutical composition according to Claim 8,
**characterized in that**
10 g of a suppository comprise:
| | |
|---|---|
| Aesculus D6 | 0.50 g |
| Dimethyl sulfoxide | 0.83 g |
| Dimethicone 350 | 0.82 g |
| Oleum cacao (cocoa butter) | 4.93 g |
| 50:50 cetyl alcohol and stearyl alcohol | 1.15 g |
| Polysorbate 80 | 0.31 g |
| Glycerol | 0.98 g |
| Isotonic sodium chloride solution to | 10.00 g. |

10. Pharmaceutical composition according to Claim 8,
**characterized in that**
the emulsion comprises 0.01 g to 0.5 g of allicin, based on 10 g of the pharmaceutical composition.

11. Pharmaceutical composition according to Claim 10,
**characterized in that**
10 g of a suppository comprise:
| | |
|---|---|
| Allicin | 0.15 g |
| Dimethyl sulfoxide | 0.83 g |
| Dimethicone 350 | 0.82 g |
| Oleum cacao (cocoa butter) | 4.93 g |
| 50:50 cetyl alcohol and stearyl alcohol | 1.15 g |
| Polysorbate 80 | 0.31 g |
| Glycerol | 0.98 g |
| Isotonic sodium chloride solution to | 10.00 g. |

12. Pharmaceutical composition comprising an emulsion according to one of Claims 1 to 4 and which is suitable for topical administration.

13. Pharmaceutical composition according to Claim 12, which is suitable for preparation of a pharmaceutical product in the form of an ointment.

## Revendications

1. Émulsion pour une utilisation pharmaceutique, l'émulsion étant constituée par des petites billes d'émulsion dont au moins 99% présentent à chaque fois un diamètre qui est inférieur ou égal à une valeur D₉₉ ≤ 3,5 µm, déterminé par la méthode de la diffractométrie de rayon laser, contenant
- un principe actif en une proportion de 0,001 à 50% en poids,
- 0,01% à 30% de polydiméthylsiloxanes, qui présentent un degré de polymérisation de n = 20 à 400,
- 0,01% à 15% d'un émulsifiant complexe présentant une valeur BHL de 9,16,
- 45% à 99% d'une solution saline biocompatible,
- 0,01% à 10% de diméthylsulfoxyde et
- 0% à 1% de chélatant,
le principe actif étant choisi parmi les principes actifs/groupes de principes actifs procaïne, agents acidothérapeutiques, agents analeptiques/antihypoxémiques, agents analgésiques/antirhumatismaux, agents antiallergiques, agents antianémiques, agents antiarhythmiques, agents antibiotiques/antiinfectieux, agents antidémence, agents antidiabétiques, antidotes, agents antiépileptiques, agents antihémorragiques, agents antihypertoniques, agents antihypoglycémiants, agents antihypotoniques, agents anticoagulants, agents antimycotiques, agents antiphlogistiques, agents antitussifs/expectorants, agents artérioscléreux, agents balnéothérapeutiques et agents pour la thérapie par la chaleur, bloquants des récepteurs bêta et du canal calcique et inhibiteurs du système rénine-angiotensine-aldostérone, agents broncholytiques/antiasthmatiques, agents thérapeutiques cholagogues et de la voie biliaire, agents cholinergiques, corticoïdes, agents à usage dermatologique, agents diurétiques, agents favorisant la circulation sanguine, inhibiteurs enzymatiques, préparations pour déficits enzymatiques et protéines de transport, agents fibrinolytiques, agents gériatriques, agents antigoutte, agents antigrippaux et agents contre les maladies dues à un refroidissement, agents gynécologiques, agents contre les hémorroïdes, agents hépatiques, agents hypnotiques/sédatifs, agents hormonaux de l'hypophyse et de l'hypothalamus, autres peptides régulateurs et leurs inhibiteurs, agents immunomodulateurs, agents cardiaques, agents contre les caries, agents parodontaux et autres préparations dentaires, agents coronaires, agents hypolipémiants, agents anesthésiques locaux/thérapeutiques neuronaux, agents gastro-intestinaux, agents antimigraineux, préparations de substances minérales, agents thérapeutiques pour la bouche et la gorge, relaxants et réverseurs musculaires, agents ophtalmiques, agents anti-ostéoporose/régulateurs du métabolisme calcique/des os, agents otologiques, agents antiparkinsoniens et autres agents contre les troubles extrapyramidaux, agents psychopharmaceutiques, agents rhinologiques/contre la sinusite, agents fortifiants/toniques, agents thérapeutiques pour la thyroïde, sérums, immunoglobulines et vaccins, hormones sexuelles et leurs inhibiteurs, agents spasmolytiques/anticholinergiques, inhibiteurs de l'agrégation des thrombocytes, agents antituberculeux, agents d'allassothérapie, agents urologiques, agents thérapeutiques pour les veines, vitamines, agents de traitement des plaies et des cicatrices, agents cytostatiques, autres agents antinéoplasiques et agents de protection et produits homéopathiques/séries de préparations enregistrés.

2. Émulsion selon la revendication 1, **caractérisée en ce que** les émulsifiants complexes sont constitués par des alcools aliphatiques présentant les longueurs de chaîne C10 à C100, des esters d'acide gras de sorbitol et/ou de glycérol et des polysorbates.

3. Émulsion selon l'une quelconque des revendications 1 à 2, **caractérisée en ce qu'**elle contient en outre des alcools polyvalents, solubles dans l'eau, de préférence du glycérol, en une concentration de 0,01% à 10%.

4. Émulsion selon l'une quelconque des revendications 1 à 3, **caractérisée en ce que** la proportion d'émulsifiants complexes dans l'émulsion n'est pas supérieure à 3%.

5. Procédé pour la préparation d'une émulsion contenant un principe actif selon l'une quelconque des revendications 1 à 4, comprenant les étapes de procédé :
a) pesée séparée du principe actif et du chélatant ;
b) pesée et fusion d'un mélange des proportions souhaitées de polydiméthylsiloxanes, de diméthylsulfoxyde et des composants de l'émulsifiant complexe dans un premier récipient à 55°C en une masse fondue crémeuse ;
c) pesée, préparation et chauffage d'une solution de chlorure de sodium et refroidissement consécutif de la solution de chlorure de sodium à une température dans la plage de température de 45°C à 55°C dans un deuxième récipient ;
d) addition du principe actif ou d'un composé soluble dans l'eau du principe actif ainsi qu'addition du chélatant dans le deuxième récipient, les deux composants étant amenés en solution ;
e) rassemblement des contenus du premier récipient et du deuxième récipient ;
f) homogénéisation continue des solutions rassemblées du premier récipient et du deuxième récipient pendant au moins cinq minutes dans un homogénéisateur à rotor-stator dans une plage de vitesses de rotation d'au moins 17.000 tours par minute et décantation consécutive d'une couche de mousse formée par l'homogénéisation ;
g) transvasement de l'ensemble du mélange dans des récipients de transvasement qui sont autoclavés à une température dans la plage entre 110 et 125°C et
h) nouvelle homogénéisation du mélange par agitation à froid des récipients de transvasement dans une machine d'agitation.

6. Procédé selon la revendication 5, **caractérisé par** les étapes de procédé :
a) pesée séparée du principe actif et de CaNa₂EDTA comme chélatant ;
b) pesée et fusion d'un mélange de diméticone, de diméthylsulfoxyde, de trioléate de sorbitane, d'alcool cétylstéarylique, de polysorbate 80, de glycérol dans un premier récipient à 55°C en une masse fondue crémeuse ;
c) préparation et chauffage d'une solution de chlorure de sodium et refroidissement consécutif de la solution de chlorure de sodium à une température de 50°C dans un deuxième récipient ;
d) addition de dichlorhydrate de procaïne ainsi qu'addition de CaNa₂EDTA comme chélatant dans le deuxième récipient, les deux composants étant amenés en solution ;
e) rassemblement des contenus du premier récipient et du deuxième récipient ;
f) homogénéisation continue des solutions rassemblées du premier récipient et du deuxième récipient pendant au moins cinq minutes dans un homogénéisateur à rotor-stator à une vitesse de rotation de 17.000 tours par minute et décantation consécutive d'une couche de mousse formée par l'homogénéisation ;
g) transvasement de l'ensemble du mélange dans des flacons à sertir qui sont autoclavés à une température de 121°C et
h) nouvelle homogénéisation du mélange par agitation à froid des flacons à sertir dans une machine d'agitation.

7. Composition pharmaceutique qui contient une émulsion selon l'une quelconque des revendications 1 à 4 et qui convient pour l'administration par voie orale, rectale ou vaginale.

8. Composition pharmaceutique selon la revendication 7, qui convient pour la préparation d'un agent pharmaceutique sous forme d'un comprimé, d'une capsule ou d'un suppositoire.

9. Composition pharmaceutique selon la revendication 8, **caractérisée en ce que** 10 g d'un suppositoire contiennent :
| | |
|---|---|
| Aesculus D6 | 0,50 g |
| Diméthylsulfoxyde | 0,83 g |
| Diméticone 350 | 0,82 g |
| Oleum Cacao (beurre de cacao) | 4,93 g |
| 50:50 alcool cétylique et alcool stéarylique | 1,15 g |
| Polysorbate 80 | 0,31 g |
| Glycérol | 0,98 g |
| Solution isotonique de chlorure de sodium ad | 10,00 g |

10. Composition pharmaceutique selon la revendication 8, **caractérisée en ce que** l'émulsion, par rapport à 10 g de la composition pharmaceutique, contient 0,01 g à 0,5 g d'allicine.

11. Composition pharmaceutique selon la revendication 10, **caractérisée en ce que** 10 g d'un suppositoire contiennent :
| | |
|---|---|
| Allicine | 0,15 g |
| Diméthylsulfoxyde | 0,83 g |
| Diméticone 350 | 0,82 g |
| Oleum Cacao (beurre de cacao) | 4,93 g |
| 50:50 alcool cétylique et alcool stéarylique | 1,15 g |
| Polysorbate 80 | 0,31 g |
| Glycérol | 0,98 g |
| Solution isotonique de chlorure de sodium ad | 10,00 g |

12. Composition pharmaceutique qui contient une émulsion selon l'une quelconque des revendications 1 à 4 et qui convient pour l'administration topique.

13. Composition pharmaceutique selon la revendication 12, qui convient pour la préparation d'un agent pharmaceutique sous forme d'une pommade.
